# EUROPEAN PATENT APPLICATION

(11) **EP 4 644 535 A1**
(43) Date of publication of application: **05.11.2025**
(21) Application number: 23912216.1
(22) Date of filing: 27.12.2023
(51) Int. Cl.: C12N 5/071, C07K 1/14, C07K 14/47, C12N 1/02

(54) **POROUS CARRIER, METHOD FOR AQUIRING EXTRACELLULAR VESICLE, METHOD FOR PRODUCING EXTRACELLULAR VESICLE, AND KIT**

(30) Priority: 28.12.2022 JP 2022212294
(71) Applicant: FUJIFILM Corporation, Tokyo 106-8620 (JP)
(72) Inventor: ISHIDOME, Takamasa, Tokyo 106-8620 (JP)
(74) Representative: Hoffmann Eitle
(86) International application number: PCT/JP2023/046845
(87) International publication number: WO 2024/143438

(57) **Abstract**

An object of the present invention is to provide a porous carrier that is capable of being acquired by separating extracellular vesicles with high purity according to the size and affinity, a method for acquiring extracellular vesicles using the porous carrier, a method for producing extracellular vesicles using the porous carrier, and a kit for acquiring extracellular vesicles, which contains the porous carrier. According to the present invention, there are provided a porous carrier that is for acquiring extracellular vesicles and has an exclusion limit molecular weight of 800 to 60,000 kDa, to which a substance having an affinity for extracellular vesicles is bound, a method for acquiring extracellular vesicles using the porous carrier, a method for producing extracellular vesicles using the porous carrier, and a kit for acquiring extracellular vesicles.

## Description

### Technical Field

The present invention relates to a porous carrier, a method for acquiring extracellular vesicles, a method for producing extracellular vesicles, and a kit.

### Background Art

In recent years, the usefulness of extracellular vesicles (EVs) has been shown for various purposes such as diagnosis and treatment. For example, it is known that nucleic acids such as proteins and microRNAs are present inside the particles of the extracellular vesicles and the extracellular vesicles are responsible for the transfer of substances between cells. Extracellular vesicles are also secreted in body fluids such as blood, and proteins, microRNAs, and the like in the extracellular vesicles have attracted attention as diagnostic markers for diseases. In addition, the applications to medical treatment, such as the anti-inflammatory effect of extracellular vesicles derived from mesenchymal stem cells and drug delivery using artificially modified extracellular vesicles, are also attracting attention.

Here, various studies have been performed so far on a method for acquiring extracellular vesicles and a method for generating extracellular vesicles.

For example, Patent Document 1 describes a carrier (Tim carrier) to which a protein (Tim protein) selected from a T cell immunoglobulin-mucin domain-containing molecule 4 (Tim4) protein, a T cell immunoglobulin-mucin domain-containing molecule 3 (Tim3) protein, or a T cell immunoglobulin-mucin domain-containing molecule 1 (Tim1) protein is bound, and a method for acquiring extracellular membrane vesicles or viruses in a specimen, the method including: (1) a step of forming a complex of the Tim protein bound to the carrier and the extracellular membrane vesicles or viruses in the specimen in the presence of calcium ions (a complex formation step); (2) a step of separating the complex from the sample (a complex separation step); and (3) a step of separating the extracellular membrane vesicles or viruses from the complex to acquire the extracellular membrane vesicles or viruses (an acquisition step).

Patent Document 2 describes a cancer diagnosis device including an extracellular vesicle capture unit that includes an immobilization carrier in which one or two or more types of lectins capable of specifically binding to a surface sugar chain of an extracellular vesicle derived from a cancer cell are immobilized, the immobilized carrier being provided to correspond to each of one or two or more types of the surface sugar chains, and captures the extracellular vesicle by a specific binding between the surface sugar chain and the lectin, and a detection unit for detecting a microRNA contained in the extracellular vesicle.

### Prior Art Documents

### Patent Documents

Patent Document 1: WO2016/088689A
Patent Document 2: JP2018-191636A

### SUMMARY OF THE INVENTION

### Object to be solved by the invention

In the method for acquiring extracellular vesicles, a method of efficiently separating and purifying extracellular vesicles with high purity is required.

For example, as a method of separating and purifying extracellular vesicles, it is known to carry out two or more of tangential flow filtration (TFF), size exclusion chromatography (SEC), and ion exchange in combination. However, according to such an aspect, there is an issue that the number of steps increases and the cost such as time and cost increases.

An object of the present invention is to provide a porous carrier that is capable of being acquired by separating extracellular vesicles with high purity according to the size and affinity, a method for acquiring extracellular vesicles using the porous carrier, a method for producing extracellular vesicles using the porous carrier, and a kit for acquiring extracellular vesicles.

### Means for solving the object

Representative aspects of the present invention are shown below. However, the present invention is not limited thereto.
<1> A porous carrier that is for acquiring extracellular vesicles and has an exclusion limit molecular weight of 800 to 60,000 kDa, to which a substance having an affinity for extracellular vesicles is bound.
<2> The porous carrier according to <1>, in which the exclusion limit molecular weight is 1,500 to 35,000 kDa.
<3> The porous carrier according to <1> or <2>, in which pores included in the porous carrier is non-penetrating pores.
<4> The porous carrier according to any one of <1> to <3>, in which a median diameter of pores included in the porous carrier is 17 to 21 nm.
<5> The porous carrier according to any one of <1> to <4>, in which the substance having an affinity for extracellular vesicles is a phosphatidylserine-binding substance.
<6> The porous carrier according to <5>, in which the phosphatidylserine-binding substance is a Tim protein.
<7> The porous carrier according to any one of <1> to <6>, in which the porous carrier is a porous particle or a porous membrane.
<8> The porous carrier according to any one of <1> to <7>, in which a binding amount of the substance having an affinity for extracellular vesicles with respect to 1 g of the porous carrier is 1,000 µg/g or less.
<9> The porous carrier according to any one of <1> to <8>, in which the porous carrier is composed of polysaccharides.
<10> The porous carrier according to any one of <1> to <9>, in which a proportion of a quantity of extracellular vesicles having a particle diameter of 200 nm or less is 85% or more in the entire acquired extracellular vesicles.
<11> A method for acquiring extracellular vesicles in a specimen using the porous carrier according to any one of <1> to <10>.
<12> A method for producing extracellular vesicles, comprising:
   bringing a specimen containing extracellular vesicles into contact with the porous carrier according to any one of <1> to <10> to form a complex of a substance having an affinity for extracellular vesicles bound to the porous carrier and the extracellular vesicles in the specimen;
   separating the complex from the specimen; and
   separating the extracellular vesicles from the complex to acquire the extracellular vesicles.
<13> A method for producing extracellular vesicles, comprising:
   bringing a specimen containing extracellular vesicles into contact with the porous carrier according to any one of <1> to <10> to form a complex of a substance having an affinity for extracellular vesicles bound to the porous carrier and the extracellular vesicles in the specimen; and
   removing the complex.
<14> A kit for acquiring extracellular vesicles, comprising:
   a porous carrier having an exclusion limit molecular weight of 800 to 60,000 kDa; and
   a substance having an affinity for extracellular vesicles.

### Effect of the invention

According to the present invention, a porous carrier that is capable of being acquired by separating extracellular vesicles with high purity according to the size and affinity, a method for acquiring extracellular vesicles using the porous carrier, a method for producing extracellular vesicles using the porous carrier, and a kit for acquiring extracellular vesicles are provided.

### EMBODIMENTS FOR CARRYING OUT THE INVENTION

### (Porous carrier)

The porous carrier according to the embodiment of the present invention is a porous carrier that is for acquiring extracellular vesicles and has an exclusion limit molecular weight of 800 to 60,000 kDa, to which a substance having an affinity for extracellular vesicles is bound.

According to the porous carrier according to the embodiment of the present invention, extracellular vesicles can be separated and acquired with high purity according to the size.

Specifically, the porous carrier according to the embodiment of the present invention is a porous carrier having an exclusion limit molecular weight of 800 to 60,000 kDa. It is considered that this porous carrier has a large number of micropores to achieve the above-described exclusion limit molecular weight.

It is considered that in a case where such a porous carrier is brought into contact with extracellular vesicles (EVs), the extracellular vesicles enter the micropores. In addition, it is considered that the substance having an affinity for the EV is more bound to the inside of the micropores having a large surface area, and it is considered that the EV binds to the substance in the micropores and to be likely to remain in the micropores.

Here, it is considered that the EVs having a small particle diameter are more likely to enter the micropores than the EVs having a large particle diameter, and are more likely to be bound to a substance having an affinity for the EVs. Therefore, it is considered that in a case where the porous carrier according to the embodiment of the present invention is brought into contact with the specimen containing EVs to form a complex of the porous carrier and EVs, and then the complex is separated from the specimen, EVs having a large particle diameter is contained on the specimen side and EVs having a small particle diameter is contained on the complex side. Thereafter, by separating EVs from the complex, EVs having a small particle diameter can be separated and acquired from a specimen containing EVs having a large particle diameter.

Here, for example, in the method according to WO2016/088689A, EVs can be recovered by using a carrier to which a Tim protein is bound.

However, for example, in the method according to WO2016/088689A, to separate the recovered EV according to the size, it is necessary to further perform size fractionation using a method such as SEC.

In a case of using the porous particles according to the embodiment of the present invention, since the recovery of EVs and the separation according to the size can be performed as one step, the loss of EVs is reduced and the separation and purification with excellent purification efficiency can be performed, as compared with a method of further performing the separation using SEC after the recovery of EVs.

It is preferable that the purification efficiency is excellent as described above, since it is possible to minimize damage to EVs in addition to suppression of time, cost, and the like.

In addition, in the method according to JP2018-191636A, by using an immobilization carrier in which a lectin capable of specifically binding to a surface sugar chain of extracellular vesicles derived from cancer cells is immobilized, EVs can be recovered with high sensitivity and specificity.

However, JP2018-191636A does not describe that the EV is recovered and acquired.

Hereinafter, the porous carrier according to the embodiment of the present invention will be described in detail. In the present invention, mol/l is also described as M. Similarly, "mmol/l" and "umol/l" are also described as "mM" and "µM".

In addition, in the present invention, "room temperature" is 23°C unless otherwise specified.

In addition, in the present invention, a combination of preferable aspects is a more preferable aspect.

### <Porous carrier>

The porous carrier according to the embodiment of the present invention is a porous carrier having an exclusion limit molecular weight of 800 to 60,000 kDa, to which a substance having an affinity for extracellular vesicles is bound.

### [Substance having affinity for extracellular vesicles]

The substance having an affinity for extracellular vesicles is not particularly limited, and examples thereof include a phosphatidylserine-binding substance such as a Tim protein and annexin V, an antibody that binds to a tetraspanin present on the surface of vesicles, such as an anti-CD9 antibody, an anti-CD63 antibody, and an anti-CD81 antibody.

Among these, the substance having an affinity for extracellular vesicles is preferably a phosphatidylserine-binding substance and more preferably a Tim protein.

As the Tim protein, a Tim4 protein, a Tim3 protein, or a Tim1 protein is preferable, and a Tim4 protein is more preferable.

The amount of the substance having an affinity for extracellular vesicles with respect to the mass of the porous carrier is preferably 1,000 µg/g or less, more preferably 30 to 1,000 µg/g, and still more preferably 30 to 800 µg/g.

### [Porous carrier]

The exclusion limit molecular weight of the porous carrier is preferably 1,000 kDa or more, more preferably 1,500 kDa or more, still more preferably 10,000 kDa or more, and particularly preferably 29,000 kDa or more.

In addition, the exclusion limit molecular weight is preferably 40,000 kDa or less, more preferably 35,000 kDa or less, still more preferably 31,000 kDa or less, and particularly preferably 30,000 kDa or less.

Furthermore, the exclusion limit molecular weight is not limited to the above-described preferred range and may be appropriately set according to the desired particle diameter of the extracellular vesicles.

As the exclusion limit molecular weight, a catalog value described for a carrier (hereinafter, also referred to as an "unbound carrier") before binding a substance having an affinity for extracellular vesicles can be used.

In a case where there is no catalog value, the exclusion limit molecular weight can be obtained, for example, by the following method.

In a case where a protein (hereinafter, also referred to as an "input") having a known molecular weight and concentration, for example, thyroglobulin (molecular weight: 669,000), ovalbumin (molecular weight: 43,000), or the like flows through the unbound carrier filled in a column at a flow rate indicating the pressure at which the carrier can withstand, for example, 60 cm/h, in the same volume as the unbound carrier, a concentration of a fraction (hereinafter, also referred to as a "retained fraction") retained in the unbound carrier is calculated by subtracting a concentration of a fraction (hereinafter, also referred to as a "unretained fraction") that has passed through the unbound carrier without being retained from the concentration of "input" (concentration of "input" - concentration of "unretained fraction"). Next, the retention rate (retention rate = "retained fraction"/"input") which is a proportion of the "retained fraction" to the "input" is calculated. Furthermore, the "retention rate" of a plurality of proteins having different molecular weights is calculated, four or more points are plotted on a two-dimensional graph in which the X axis represents the molecular weight and the Y axis represents the retention rate, and an approximate straight line is drawn. The exclusion limit molecular weight is obtained by calculating the molecular weight at which the "retention rate" of the approximate straight line is 0.

The pores included in the porous carrier according to the embodiment of the present invention may be penetrating pores or non-penetrating pores, but are preferably non-penetrating pores.

In addition, the median diameter of the pores included in the porous carrier according to the embodiment of the present invention is preferably 17 to 21 nm, more preferably 18 to 21 nm, and still more preferably 19 to 21 nm.

The median diameter can be measured, for example, by a method described in Examples described later, using an unbound carrier.

The form of the porous carrier according to the embodiment of the present invention is not particularly limited, but is preferably a porous particle or a porous film, and more preferably a porous particle.

The number-average particle diameter of the porous particles is not particularly limited, but is preferably 10 nm to 100 µm and more preferably 1 µm to 100 µm.

The porous carrier can be used without particular limitation as long as it is a porous carrier having the above-described exclusion limit molecular weight, but it is preferably composed of polysaccharides, silica, addition polymerization polymers such as vinyl resins and styrene resins, and the like, and more preferably composed of polysaccharides.

As the polysaccharide, agarose or cephalose is preferable, and agarose is more preferable.

### [Method for binding substance having affinity for extracellular vesicle to unbounded carrier (immobilization method)]

The method for binding a substance having an affinity for extracellular vesicles according to the present invention to an unbound carrier according to the present invention may be according a method well known per se for binding a protein to a carrier, and examples thereof include a method for binding by affinity binding, a method for binding by chemical bonding (for example, the method described in JP3269554B, the method described in WO2012/039395A, and the method described in WO2016/088689A), a method for binding by physical adsorption (for example, the method described in JP1993-041946B (JP-H05-041946B)), and the like, and a method of binding by chemical bonding is preferable.

As a method for binding the substance having an affinity for extracellular vesicles according to the present invention to the unbound carrier according to the present invention by a chemical bond, under the condition in which the substance having an affinity for extracellular vesicles according to the present invention and the unbound carrier according to the present invention are bound to each other according to a known method per se, the substance having an affinity for extracellular vesicles according to the present invention and the unbound carrier according to the present invention are only required to be brought into contact with each other.

The reaction temperature between the substance having an affinity for extracellular vesicles according to the present invention and the unbound carrier according to the present invention is preferably 2°C to 37°C and more preferably 4°C to 11°C.

The reaction time between the substance having an affinity for extracellular vesicles according to the present invention and the unbound carrier according to the present invention is preferably 4 hours to 48 hours and more preferably 12 hours to 24 hours.

The amount of the substance having an affinity for extracellular vesicles according to the present invention, which is brought into contact with the unbound carrier according to the present invention, is 30 to 1,000 µg, and preferably 30 to 800 µg.

The chemical bond between the substance having an affinity for extracellular vesicles according to the present invention and the unbound carrier according to the present invention is preferably formed by bringing a solution containing the substance having an affinity for extracellular vesicles according to the present invention into contact with the unbound carrier according to the present invention.

The solution containing a substance having an affinity for extracellular vesicles according to the present invention may be any solution as long as it dissolves the substance having an affinity for extracellular vesicles according to the present invention in a stable state, and examples thereof include purified water and a buffer solution having a buffering action at, for example, pH 6.0 to 9.5, preferably 7.0 to 8.0 (for example, a Good's buffer such as MOPS, a phosphate buffer, a carbonate buffer, PBS, TBS, HBS, and the like). In addition, the concentration of the buffering agent in these buffer solutions is preferably 5 to 100 mM and more preferably 10 to 50 mM. The concentration in a case of adding NaCl is generally preferably 100 to 200 mM and more preferably 140 to 160 mM.

In addition, the solution may contain, for example, sugars, salts such as NaCl, surfactants, preservatives, proteins, and the like, as long as after the solution containing a substance having an affinity for extracellular vesicles according to the present invention is brought into contact with the unbound carrier according to the present invention, the amount thereof does not interfere with the binding between the unbound carrier and the extracellular vesicles.

Specific examples of the method for binding the substance having an affinity for extracellular vesicles according to the present invention to the unbound carrier according to the present invention by a chemical bond include the methods described in Examples described later. However, the present invention is not limited thereto.

In addition, after binding the substance having an affinity for extracellular vesicles to the unbound carrier as described above, a blocking treatment may be performed, which is usually performed in this field.

Furthermore, after binding the substance having an affinity for extracellular vesicles to the unbound carrier as described above, a purification treatment may be performed, which is usually performed in this field. The purification treatment may be any treatment as long as it can remove impurities adhering to the surface of the porous carrier, and the substance having an affinity for extracellular vesicles, which has weakly bound to the carrier, and examples thereof include a method of washing the porous carrier according to the embodiment of the present invention with a known washing solution such as guanidine hydrochloride.

The porous carrier can be used in an aspect in which the porous carrier is used as particles (beads) of a porous carrier, an aspect in which the porous carrier is used by being filled in a column, an aspect in which the porous carrier is used as a membrane consisting of a porous carrier, and the like.

### [Extracellular vesicles]

The porous carrier according to the embodiment of the present invention is a porous carrier for acquiring extracellular vesicles.

The extracellular vesicle is a cell-derived small membrane vesicle composed of a lipid bilayer.

Examples of the extracellular vesicle include various extracellular vesicles classified according to their origin and size of small membrane vesicles, as described in Nature Reviews Immunology 9, 581-593 (August 2009), "Obesity Research" Vol. 13 No. 2 2007, topics by Naoto Aoki et al., or the like. Specific examples thereof include exosomes, microvesicles, ectosomes, membrane particles, exosome-like vesicles, apoptotic vesicles, adiposomes, and the like.

The exosome is a small membrane vesicle derived from a late endosome, which is composed of a lipid bilayer membrane and has phosphatidylserine on the membrane surface. The diameter of the exosome is preferably 50 nm to 200 nm, more preferably 50 nm to 150 nm, and still more preferably 50 nm to 100 nm. It is known that exosomes contain proteins such as tetraspanins such as CD63 and CD9, Alix, TSG101, Lamp-1, and flotillin.

The microvesicle is a small membrane vesicle derived from a plasma membrane, which is composed of a lipid bilayer and has phosphatidylserine on the surface of the membrane. The diameter of the microvesicle is preferably 100 nm to 1,000 nm, more preferably 100 nm to 800 nm, and still more preferably 100 nm to 500 nm. It is known that the microvesicles contain proteins such as integrin, selectin, and CD40 ligand.

The ectosome is a small membrane vesicle derived from a plasma membrane, which is composed of a lipid bilayer and has phosphatidylserine on the membrane surface. The diameter of the ectosome is preferably 50 nm to 200 nm, more preferably 50 nm to 150 nm, and still more preferably 50 nm to 100 nm. It is known that the ectosome contains CR1 and a proteolytic enzyme but does not contain CD63.

The membrane particle is a small membrane vesicle derived from a plasma membrane, which is composed of a lipid bilayer and has phosphatidylserine on the membrane surface. The diameter of the membrane particles is preferably 50 nm to 80 nm. It is known that the membrane particle contains CD133 but do not contain CD63.

The exosome-like vesicle is a small membrane vesicle derived from an early endosome, which is composed of a lipid bilayer membrane and has phosphatidylserine on the membrane surface. The diameter of the exosome-like vesicle is preferably usually 20 nm to 50 nm. It is known that the exosome-like vesicle contains TNFRI.

The apoptotic vesicle is a small membrane vesicle derived from an apoptotic cell, which is composed of a lipid bilayer membrane and has phosphatidylserine on the membrane surface. The diameter of the apoptotic vesicle is preferably 50 nm to 500 nm, more preferably 50 nm to 300 nm, and still more preferably 50 nm to 200 nm. It is known that the apoptotic vesicle contains histones.

The adiposome is a small membrane vesicle derived from an adipocyte, which is composed of a lipid bilayer membrane and has phosphatidylserine on the membrane surface. The diameter of the adibosome is preferably 100 nm to 1,000 nm, more preferably 100 nm to 800 nm, and still more preferably 100 nm to 500 nm. It is known that adiposomes contain milk fat globule-EGF factor 8 (MFG-E8).

Here, according to the porous carrier according to the embodiment of the present invention, extracellular vesicles can be separated and acquired with high purity according to the size and affinity.

For example, by setting the exclusion limit molecular weight and the like in the porous carrier according to the embodiment of the present invention such that extracellular vesicles having a particle diameter of 200 nm or less can be retained in the pores, it is possible to selectively acquire extracellular vesicles having a particle diameter of 200 nm or less and being capable of binding to an affinity substance from a specimen containing extracellular vesicles, and to selectively remove extracellular vesicles having a particle diameter of 200 nm or less and being capable of binding to an affinity substance from a specimen containing extracellular vesicles.

According to the porous carrier according to the embodiment of the present invention, extracellular vesicles can be easily separated and acquired from a large amount of specimens with high purity according to the size and affinity.

**In** the entire extracellular vesicles acquired by the porous carrier according to the embodiment of the present invention, the proportion of the quantity of extracellular vesicles having a particle diameter of 200 nm or less is preferably 85% or more, more preferably 88% or more, and still more preferably 90% or more.

The upper limit of the above-described proportion is not particularly limited, and may be 100%.

### (Method for acquiring extracellular vesicles)

The method for acquiring extracellular vesicles according to the embodiment of the present invention is a method for acquiring extracellular vesicles in a specimen using the porous carrier according to the embodiment of the present invention.

Specifically, the method for acquiring extracellular vesicles according to the embodiment of the present invention preferably includes bringing a specimen into contact with the porous carrier according to the embodiment of the present invention to form a complex of a substance having an affinity for extracellular vesicles bound to the porous carrier and the extracellular vesicles in the specimen (a complex formation step), separating the complex from the specimen (a complex separation step), and separating the extracellular vesicles from the complex to acquire the extracellular vesicles (an acquisition step).

### <Complex formation step>

### [Specimen]

The specimen according to the present invention may be any of a specimen containing the extracellular vesicles according to the present invention in a liquid or a specimen that may contain the extracellular vesicles according to the present invention. The specimen according to the present invention may be any of a specimen derived from a living body or a specimen in which the extracellular vesicles according to the present invention are suspended in a solution such as a culture medium or a buffer solution. Specific examples of the specimen according to the present invention include body fluids such as blood, saliva, urine, milk, amniotic fluid, and ascites, cell culture supernatants, and the like.

The above-described liquid may be any liquid as long as it allows the extracellular vesicles according to the present invention to be suspended in a stable state and does not cause an interfere with the formation of a complex with extracellular vesicles in a specimen due to the binding to a porous carrier, and examples thereof include water and a buffer solution (for example, TBS, HBS, or the like) having a buffering action at a pH of 7.0 to 8.0, preferably 7.2 to 7.6. As the buffer solution, a buffer solution that is less likely to cause precipitation by binding to calcium is preferable. In addition, the concentration of the buffering agent in these buffer solutions is preferably 5 to 50 mM and more preferably 10 to 30 mM. The concentration of NaCl is preferably 100 to 200 mM and more preferably 140 to 160 mM.

This solution may contain, for example, sugars, salts such as NaCl, surfactants, preservatives, proteins, and the like. Examples of the surfactants include Tween 20 and the like, and the concentration of the surfactant is preferably 0.00001% to 0.2% and more preferably 0.0005% to 0.1%.

### [Formation of complex]

For example, in a case where the substance having an affinity for extracellular vesicles is a Tim protein, the formation of the complex is preferably performed in the presence of calcium ions.

The concentration of the calcium ion in a case of bringing the specimen according to the embodiment of the present invention into contact with the porous carrier according to the present invention is preferably 0.5 to 100 mM, more preferably 1.0 to 10 mM, and still more preferably 2.0 to 5.0 mM.

It is preferable that until the complex between the porous carrier according to the embodiment of the present invention and the extracellular vesicles in the specimen according to the present invention are formed and the complex is subjected to the complex separation step, the solution containing the complex contains calcium ions at the above-described concentration.

The origin of the calcium ion is not particularly limited, and examples thereof include calcium chloride, calcium hydroxide, calcium hydrogen carbonate, calcium iodide, calcium bromide, calcium acetate, and the like. Calcium chloride, calcium hydrogen carbonate, or calcium iodide is preferable, and calcium chloride or calcium hydrogen carbonate is more preferable.

In a case of bringing the porous carrier according to the embodiment of the present invention into contact with the specimen, as a method of allowing calcium ions to be contained, it is only required to contain calcium ions in at least one of the solution containing the porous carrier according to the embodiment of the present invention or the specimen such that the concentration of calcium ions in a case of bringing the porous carrier into contact with the specimen is within the above-described range. In addition, a solution containing calcium ions, a solution containing the porous carrier according to the embodiment of the present invention, and a specimen may be mixed such that the concentration of the calcium ions in a case of bringing the porous carrier into contact with the specimen is within the above-described range.

The liquid in the solution containing calcium ions is the same as the liquid in the specimen described above, and the same applies to the specific examples and the like.

In a case where the porous carrier according to the embodiment of the present invention is brought into contact with a specimen, the porous carrier may be used in a state of being filled in a housing. For example, a complex can be formed by filling the porous carrier according to the embodiment of the present invention in a glass or plastic column and bringing the porous carrier into contact with the specimen by a device or a system such as a peristaltic pump or HPLC.

### [Amount of specimen]

In the complex formation step, the amount of the specimen being brought into contact with 1 mg of the porous particles according to the embodiment of the present invention is preferably 0.01 to 10 ml, more preferably 0.01 to 1 ml, and still more preferably 0.01 to 0.5 ml.

### [Temperature]

In the complex formation step, the temperature at the time of bringing the specimen into contact with the porous carrier according to the embodiment of the present invention is preferably 4°C to 37°C, more preferably 4°C to 30°C, and still more preferably 15°C to 30°C.

### [Time]

In the complex formation step, the contact time between the specimen and the porous carrier according to the embodiment of the present invention is preferably 0.5 to 24 hours, more preferably 0.5 to 8 hours, and still more preferably 0.5 to 4 hours in the batch method. In the column method, the contact time is preferably 1 to 5 minutes, more preferably 1 to 3 minutes, and still more preferably 1 to 2 minutes.

### <Complex separation step>

**In** the complex separation step, for example, the complex and a specimen other than the complex can be separated by subjecting a solution containing the complex to a centrifugal treatment.

Examples thereof include a method in which a complex is loaded on a filter and subjected to a centrifugal treatment to separate a specimen other than the complex as a residual liquid.

The centrifugation conditions may be determined in consideration of the particle diameter, the density, and the like of the porous particles, but may be set to, for example, conditions of 1 to 1,000 × g for 30 seconds to 30 minutes.

**In** addition, for example, the complex and a specimen other than the complex may be separated by subjecting a solution containing the complex to a column treatment.

Examples thereof include a method in which a solution containing a complex is loaded into a column, and then a specimen other than the complex is separated as a residual liquid by, for example, natural dropping or pressure by a peristaltic pump.

The complex may be washed with a calcium ion-containing washing solution before the centrifugal treatment or the column treatment. The concentration of the calcium ion in the calcium ion-containing washing solution and the liquid are the same as the concentration of the calcium ion and the liquid in the specimen in the above-described complex formation step.

### <Acquisition step>

Examples of the acquisition step include a method of using a protein denaturant and a method of reducing the concentration of calcium ions.

Examples of the method of using the protein denaturant include the methods described in paragraphs 0162 to 0169 of WO2016/088689A.

Specifically, as a method of reducing the concentration of calcium ions, the concentration of the calcium ions in the solution containing the complex according to the present invention is preferably set to less than 0.5 mM, more preferably set to less than 0.4 mM, and still more preferably set to less than 0.2 mM.

Examples of the method of reducing the concentration of the calcium ions include a method of using a calcium ion chelating agent, a method of using a solution that does not contain calcium ions, and the like.

As a method of using a solution containing no calcium ions, the method described in paragraphs 0174 to 0191 of WO2016/088689A can be referred to.

### [Method of using calcium ion chelating agent]

The calcium ion chelating agent may be any compound as long as it is a compound capable of chelating calcium ions, and examples thereof include ethylenediaminetetraacetic acid (EDTA), nitrilotriacetic acid (NTA), diethylenetriaminepentaacetic acid (DTPA), L-glutamic acid diacetic acid (GLDA), hydroxyethylethylenediaminetriacetic acid (HEDTA), ethylene glycol bis(β-aminoethylether)-N,N,N,N-tetraacetic acid (GEDTA), triethylenetetramine-N,N,N',N",N‴,N‴-hexaacetic acid (TTHA), N-(2-hydroxyethyl)iminodiacetic acid (HIDA), N,N-bis(2-hydroxyethyl)glycine (DHEG), and trans-1,2-Diaminocyclohexane-N,N,N',N'-tetraacetic acid, monohydrate (CyDTA), and EDTA, GEDTA, or CyDTA is preferable.

To action of the calcium ion chelating agent on the calcium ion bound to the complex according to the present invention is generally performed by bringing a solution containing the calcium ion chelating agent (hereinafter, may be abbreviated as "calcium ion chelating agent-containing solution") into contact with the complex according to the present invention to react the calcium ion bound to the complex according to the present invention with the calcium ion chelating agent in the calcium ion chelating agent-containing solution.

The contact between the calcium ion chelating agent-containing solution and the complex according to the present invention can be performed, for example, by a method for suspending the complex in the solution.

### -Calcium ion chelating agent-containing solution-

The solution containing a calcium ion chelating agent may be any solution as long as the calcium ion chelating agent is dissolved, and examples thereof include purified water, a buffer solution, and the like. As the buffer solution, for example, a buffer solution (for example, PBS, TBS, HBS, or the like) having a buffering action at a pH of 7.0 to 8.0, preferably 7.2 to 7.6 is preferable. In addition, the concentration of the buffering agent in these buffer solutions is appropriately selected from a range of usually 5 to 50 mM and preferably 10 to 30 mM, and the concentration of NaCl is appropriately selected from a range of usually 100 to 200 mM and preferably 140 to 160 mM. The calcium ion chelating agent-containing solution may contain, for example, sugars, salts such as NaCl, a preservative, a protein, and the like.

The concentration of the calcium ion chelating agent in the calcium ion chelating agent-containing solution is, for example, preferably 0.5 to 500 mM, more preferably 0.5 to 100 mM, and still more preferably 0.5 to 50 mM.

The pH of the calcium ion chelating agent-containing solution is preferably 6.0 to 9.0, more preferably 7.0 to 8.0, and still more preferably 7.2 to 7.6.

The amount of the calcium ion chelating agent-containing solution may be any amount as long as the concentration of calcium ions is less than the effective concentration and extracellular vesicles are separated from the complex according to the present invention.

### -Action (contact) conditions-

The temperature and time for the action (contact) of the complex with the calcium ion chelating agent is, for example, 4.0°C to 37°C, preferably 10°C to 30°C, and more preferably 20°C to 30°C, and for example, 5 to 60 minutes, preferably 10 to 30 minutes, and more preferably 15 to 25 minutes.

### (Method for producing extracellular vesicles)

A first aspect of the method for producing extracellular vesicles according to the embodiment of the present invention includes bringing a specimen containing extracellular vesicles into contact with a porous carrier to form a complex of a substance having an affinity for extracellular vesicles bound to the porous carrier and the extracellular vesicles in the specimen (a complex formation step), separating the complex from the specimen (a complex separation step), and separating the extracellular vesicles from the complex to acquire the extracellular vesicles (an acquisition step).

The details and preferable aspects of the complex formation step, the complex separation step, and the acquisition step according to the first aspect of the method for producing extracellular vesicles according to the embodiment of the present invention are the same as the details and preferable aspects of the complex formation step, the complex separation step, and the acquisition step according to the above-described method for acquiring extracellular vesicles according to the embodiment of the present invention.

According to the first aspect of the method for producing extracellular vesicles according to the embodiment of the present invention, since the extracellular vesicles that has formed a complex can be recovered, extracellular vesicles having a small particle diameter can be obtained.

Specifically, for example, the proportion of extracellular vesicles having a particle diameter of 200 nm or less in the entire extracellular vesicles obtained by the first aspect of the method for producing extracellular vesicles according to the embodiment of the present invention is preferably 85% or more, more preferably 88% or more, and still more preferably 90% or more.

The upper limit of the above-described proportion is not particularly limited, and may be 100%.

### (Method for producing extracellular vesicles)

A second aspect of the method for producing extracellular vesicles according to the embodiment of the present invention includes bringing a specimen containing extracellular vesicles into contact with the porous carrier according to the embodiment of the present invention to form a complex of a substance having an affinity for extracellular vesicles bound to the porous carrier and the extracellular vesicles in the specimen (a complex formation step), and removing the complex (a removal step).

The details and preferable aspects of the complex formation step according to the second aspect of the method for producing extracellular vesicles according to the embodiment of the present invention are the same as the details and preferable aspects of the complex formation step according to the above-described method for acquiring extracellular vesicles according to the embodiment of the present invention.

### <Removal Step>

The details of the removal step are the same as those in the above-described complex separation step, except that the target to be recovered is a specimen other than the complex, which is different from the complex.

Examples thereof include a method in which a complex is loaded on a filter and subjected to a centrifugal treatment to separate a specimen other than the complex as a residual liquid and acquire the residual liquid, a method in which a solution containing a complex is loaded on a column and a specimen other than the complex is separated as a residual liquid by natural dropping or pressure by a peristaltic pump, and the like.

According to the second aspect of the method for producing extracellular vesicles according to the embodiment of the present invention, since the extracellular vesicles that has formed a complex can be removed, extracellular vesicles having a large particle diameter can be obtained.

Specifically, for example, the proportion of extracellular vesicles having a particle diameter of 200 nm or less in the entire extracellular vesicles obtained by the second aspect of the method for producing extracellular vesicles according to the embodiment of the present invention is preferably 15% or less, more preferably 12% or less, and still more preferably 10% or less.

The lower limit of the above-described proportion is not particularly limited, and may be 0%.

### (Kit)

The kit according to the embodiment of the present invention is a kit for acquiring extracellular vesicles, the kit including a porous carrier having an exclusion limit molecular weight of 800 to 60,000 kDa, and a substance having an affinity for extracellular vesicles.

The porous carrier is preferably a porous carrier in which a substance having an affinity for extracellular vesicles is not bound (that is, an unbound carrier).

The porous carrier according to the embodiment of the present invention can be obtained by binding the unbound carrier to the substance having an affinity for extracellular vesicles.

Examples of the method for binding include the method described in the above-described immobilization method.

The preferable aspects of the porous carrier having an exclusion limit molecular weight of 800 to 60,000 kDa, the substance having an affinity for extracellular vesicles, and the porous carrier according to the embodiment of the present invention obtained by binding these are the same as the preferable aspects described in the porous carrier according to the embodiment of the present invention.

In addition, the kit according to the embodiment of the present invention may include a manual for the method for acquiring according to the embodiment of the present invention, a manual for the method for producing according to the embodiment of the present invention, and the like. The "manual" means a kit instruction manual, an attached document, a pamphlet (leaflet), or the like in which the features, principles, operation procedures, determination procedures, and the like in the above-described method are substantially described in sentences, diagrams, or the like.

In addition, the kit according to the embodiment of the present invention may include a filter for complex separation, which is used in the above-described complex separation step, a calcium ion chelating agent or a calcium ion chelating agent-containing solution, which is used in the above-described acquisition step, and the like.

### Examples

Hereinafter, the present invention will be described in detail using examples. Materials, using amounts, proportions, treatment details, treatment content, and the like shown in the following examples can be appropriately changed without departing from the gist of the present invention. Therefore, the scope of the present invention is not limited to the specific examples described below.

### (Culture of bone marrow MSC)

The bone marrow-derived MSC supernatant was obtained by the following operation.

Bone marrow MSC (Lonza #PT-2501) was proliferated and cultured in MSCulture^{™} High Growth (FUJIFILM Wako Pure Chemical Corporation #132-19345/133-19331) to which 15% bovine serum had been added at 200 µL/cm². Next, the bone marrow MSCs were seeded in the same culture medium of 200 µL/cm² at 5 × 10³ cells/cm² in a flask, cultured under the conditions of 37°C and 5% CO₂ until the cell density reached 90%, and then washed once with PBS in the same amount as the amount of the culture medium.

Next, EV-UP medium (FUJIFILM Wako Pure Chemical Corporation #053-09451/298-84001) was added thereto at 400 µL/cm², and the cells were cultured for 5 days under the conditions of 37°C and 5% CO₂. The cell supernatant was recovered, and EV-save for in vivo (FUJIFILM Wako Pure Chemical Corporation #050-09461) was added thereto to be 100 times diluted, thereby obtaining a "bone marrow-derived MSC supernatant".

### (Pretreatment of culture supernatant)

The bone marrow-derived MSC supernatant was treated as follows to prepare various pretreated supernatants.

The bone marrow-derived MSC supernatant was subjected to a centrifugal treatment at 300 × g for 10 minutes to obtain a supernatant solution, which was referred to as a "300 × g-treated supernatant sample".

The bone marrow-derived MSC supernatant was subjected to a centrifugal treatment at 2,000 × g for 20 minutes to obtain a supernatant solution, which was referred to as a "2,000 × g-treated supernatant sample".

The bone marrow-derived MSC supernatant was treated with a 0.22 µm filter (Millipore #SLGVR33RS) and referred to as a "0.22 µm filter-treated supernatant sample".

### (Preparation of Tim4-immobilized beads)

The Tim4-immobilized beads were obtained by the following operation.

WorkBeads 40/100 ACT, 40/1000 ACT, 40/10000 ACT, and 40/30000 ACT (all of which are manufactured by Bio-Works and are beads having non-penetrating pores) were prepared, and 100 µL of each of the beads was suspended in 1 mL of a phosphate buffer (50 mM, pH 7.4) to obtain a suspension. 16 µg of Tim4-Fc protein (FUJIFILM Wako Pure Chemical Corporation #137-18511) was added to each of the above-described suspensions, and the mixture was shaken (1,400 rpm) at 4°C for 24 hours. Each of the suspensions after the shaking was subjected to a centrifugal treatment at 2,000 × g for 1 minute, and the supernatant was recovered and referred to as "non-immobilized fraction". Each of the beads after the centrifugal treatment was washed with TBS three times, each of the beads after washing was resuspended in 200 µL of TBS to prepare a 50% slurry, and then incubated at room temperature for 1 hour or more to obtain beads in which Tim4 was immobilized. Hereinafter, the Tim4-immobilized beads produced using WorkBeads 40/100 ACT are also referred to as "40/100", the Tim4-immobilized beads produced using 40/1000 ACT are also referred to as "40/1000", the Tim4-immobilized beads produced using 40/10000 ACT are also referred to as "40/10000", and the Tim4-immobilized beads produced using 40/30000 ACT are also referred to as "40/30000".

### (Measurement of immobilization rate of Tim4)

The immobilization rate of Tim4 was measured by the following operation.

2 µg/mL (100 mM carbonate buffer (pH 9.5)) anti-TIM4/TIMD-4 (Millipore #MABC958) was added to each well of a 96-well plate (Thermo Fisher #445101) at 50 µL/well, and the mixture was allowed to react at 4°C overnight. After the reaction, 10 mg/mL (TBS) BlockAce (KAC #UKB80) was added to each well of the 96-well plate at 200 µL/well, the mixture was shaken at room temperature for 1 hour, and each well was washed with TBST three times. Recombinant Tim4-Fc (FUJIFILM Wako Pure Chemical Corporation #137-18511) was added to each well after washing at 0, 0.125, 0.25, 0.5, 1, 2, 4, and 8 ng/mL at 100 µL/well.

Next, the above-described "non-immobilized fraction" was diluted 100 times with TBST and added thereto at 100 µL/well. The mixture was shaken at room temperature for 1 hour and each well was washed three times with TBST. Anti-human IgG Fc-POD (Jackson Immuno Research #109-035-098) was diluted 80,000 times with TBST and added to each well at 100 µL/well, the mixture was shaken at room temperature for 1 hour, and each well was washed with TBST 5 times. Next, a TMB solution (FUJIFILM Wako Pure Chemical Corporation #208-17371) was added thereto at 100 µL/well.

After allowing the mixture to stand at room temperature for 30 minutes, 1 M (mol/L) HCl was added to each well at 100 µL/well. The absorbance was measured using Spark (Tecan). A standard curve was drawn with Recombinant Tim4-Fc (0.125, 0.25, 0.5, 1, 2, 4, 8 ng/mL), the amount of Tim4-Fc contained in each of the above-described "non-immobilized fraction" was quantified, and the immobilization rate of each of the beads was calculated according to the following expression. Immobilization rate (%) = (16 µg - (amount (µg) of Tim4-Fc contained in "non-immobilized fraction")/16 µg × 100

The immobilization rate of each bead is shown in Table 1.

**[Table 1]**

| | Immobilization rate |
|---|---|
| 40/30000 | 99% or more |
| 40/10000 | 99% or more |
| 40/1000 | 99% or more |
| 40/100 | 99% or more |

From Table 1, it can be seen that there is no change in the immobilization rate depending on the beads, and almost all Tim4/Fc are immobilized on the beads.

### (EV purification from MSC supernatant by preparing Tim4-immobilized beads)

EV purification was performed by the following operation.

1 mL of the above-described "300 × g-treated supernatant sample", "2,000 × g-treated supernatant sample", or "0.22 µm filter-treated supernatant sample" was added to 10 µL of each of three types of Tim4-immobilized beads of "40/30000", "40/10000", or "40/1000", and the mixture was mixed by inversion at room temperature for 2 hours. The beads were precipitated by centrifugal treatment at 2,000 × g for 1 minute, and the supernatant solution (the "unrecovered fraction") was recovered. Each of the precipitated beads was washed twice with 400 µL of 0.0005% Tween 20 + 2 mM CaCl₂/TBS, and then loaded on a 0.45 µm filter, and subjected to centrifugal treatment at 400 × g for 1 minute to remove the residual liquid. "An operation of adding 50 µL of 10 mM EDTA + 1% EV-save for in vivo/TBS to each bead on the filter, allowing the mixture to stand at room temperature for 10 minutes, and then recovering the eluate by a centrifugal treatment at 400 × g for 1 minute" was repeated twice, the eluates recovered twice were mixed, and the mixture was referred to as a "recovered fraction".

### (EV purification from MSC supernatant by MagCapture Exosome Isolation Kit PS)

EV purification was performed by the following operation.

Tim4-immobilized magnetic beads were prepared according to the protocol of MagCapture (registered trademark) Exosome Isolation Kit PS (manufactured by FUJIFILM Wako Pure Chemical Corporation). 1 mL of the "300 × g-treated supernatant sample", "2,000 × g-treated supernatant sample", or "0.22 µm filter-treated supernatant sample" was added to 60 µL of the obtained Tim4-immobilized magnetic bead, the mixture was mixed by inversion at room temperature for 2 hours, and then the beads were accumulated on a magnetic stand to recover the supernatant solution "unrecovered fraction". The beads were washed three times with 1 mL of 0.0005% Tween 20 + 2 mM CaCl₂/TBS. Next, "an operation of adding 50 µL of 10 mM EDTA + 1% EV-save for in vivo/TBS to the bead, allowing the mixture to stand at room temperature for 10 minutes, accumulating the beads with a magnetic stand, and recovering the supernatant solution" was repeated twice, the supernatant solutions recovered twice were mixed, and the mixture was referred to as a "recovered fraction".

### (Measurement of particle diameter and number of particles of recovered EV)

The particle diameter and the number of particles of the EV were measured by the following operation.

The "recovered fraction" obtained from the "300 × g-treated supernatant sample" among the above-described recovered fractions was measured with NANOSIGHT NS300 (Malvern Panalytical) (Camera Level: 16, Detection Threshold: 8). Using the analysis software mounted on NANOSIGHT NS300, the average value (number-average particle diameter), D₅₀ (median value), D₉₀ (particle diameter at which the proportion of particles having a particle diameter equal to or less than the particle diameter is 90%), the proportion of particles having a particle size of 200 nm or less, and the number of particles having a particle size of 200 nm or less, which were included in the "recovered fraction", were calculated and shown in Tables 2 and 3.

**[Table 2]**

| | Number-average particle diameter | D₅₀ | D₉₀ |
|---|---|---|---|
| MagCapture | 166.1 | 160.3 | 237.8 |
| 40/30000 | 145.6 | 141.3 | 202.8 |
| 40/10000 | 128.8 | 123.8 | 182.2 |
| 40/1000 | 126 | 120.3 | 178.8 |

**[Table 3]**

| | Proportion of particles having particle diameter of 200 nm or less | Number of particles having particle diameter of 200 nm or less |
|---|---|---|
| MagCapture | 79.2% | 4.1×10⁹ |
| 40/30000 | 90.0% | 2.0×10⁹ |
| 40/10000 | 95.4% | 5.0×10⁸ |
| 40/1000 | 97.7% | 1.7×10⁸ |

From Table 2, it is suggested that the smaller the exclusion limit molecular weight is, the smaller the particle diameter of the purified EV is, and the EV (hereinafter, also referred to as small EV) having a particle diameter of 200 nm or less is easily purified depending on the exclusion limit molecular weight. On the other hand, in MagCapture having no exclusion limit molecular weight, it is considered that the size removal effect due to the exclusion limit molecular weight is not exhibited because the particle diameter of the purified EV is large as compared with "40/30000", "40/10000", and "40/1000".

From Table 3, it is suggested that as the exclusion limit molecular weight decreases, the proportion of EVs having a particle diameter of 200 nm or less in the purified EVs increases, and as the exclusion limit molecular weight decreases, small EVs are more likely to be purified. On the other hand, it is considered that since in MagCapture having no exclusion limit molecular weight, the proportion of the purified EVs having a particle diameter of 200 nm or less is low as compared with "40/30000", "40/10000", and "40/1000", the size removal effect due to the exclusion limit molecular weight is not exhibited.

In addition, considering that the object of the present invention is to "efficiently separate and purify extracellular vesicles with high purity", it is considered that 40/30000 is suitable for large-scale purification because the proportion of EVs having a particle diameter of 200 nm or less is 90% or more, which is high purity, and the purification can be performed at the number of particles of 1.0 × 10⁹ or more per 1 mL of the supernatant. (Here, 1.0 × 10⁹ is converted to 1.0 × 10¹² in terms of 1 L, and in a case where the dose of the drug to be administered to a mouse (20 g) (JCI Insight. 2019 Nov 1; 4(21): e128060) is converted to a dose of the drug to be administered to a human (60 kg), it is assumed that 10¹² units are required for intravenous administration to one human. From the above, it is considered that 40/30000 is the most suitable form.) In addition, in a case where it is desired to improve the proportion of EVs having a particle diameter of 200 nm or less, 40/10000 or 40/1000 can be suitably used.

### (Measurement of particle diameter and number of particles of unrecovered fraction)

The particle diameter and the number of particles in the unrecovered fraction were measured by the following operation.

Among the "unrecovered fraction", the fraction obtained from the "300 × g-treated supernatant sample" was subjected to centrifugal treatment at 110,000 × g for 70 minutes, and the supernatant solution was removed. The precipitate was resuspended in 50 µL of PBS. For the obtained EV in the re-suspension, the number-average particle diameter, D₅₀, and D₉₀ were measured with NS300 (Malvern Panalytical) (Camera Level: 16, Detection Threshold: 8). The measurement results are shown in Table 4.

**[Table 4]**

| | Number-average particle diameter | D₅₀ | D₉₀ |
|---|---|---|---|
| 40/30000 | 168.8 | 145.8 | 292.6 |
| 40/10000 | 180.6 | 161.3 | 286.5 |
| 40/1000 | 180.1 | 151.9 | 306.2 |

In any purification using the beads, the "unrecovered fraction" includes EVs having a large particle diameter (large EV) as compared with the "recovered fraction", which suggests that large particles are removed by the size removal effect.

### (Comparison of expression of EV marker by ELISA and NTA)

The expression of the marker was compared by the following operation.

The "recovered fraction" obtained from the "2,000 × g-treated supernatant sample" among the "recovered fractions" described above was subjected to operation using a PS Capture Exosome ELISA kit (Streptavidin HRP) (FUJIFILM Wako Pure Chemical Corporation #298-80601) according to the protocol, and absorbance was measured with a plate reader (Spark #Tecan) to obtain a CD63 signal. In addition, the number of particles of the "recovered fraction" obtained from the "2,000 × g-treated supernatant sample" among the above-described "recovered fractions" was measured (Camera Level: 16, Detection Threshold: 8) with NS300 (Malvern Panalytical), and the particle concentration (× 10¹⁰ particles/mL) was calculated. The obtained CD63 signal was divided by the calculated particle concentration (× 10¹⁰ particles/mL) to calculate the CD63 signal corrected by the number of particles.

For the CD63 signal corrected by the calculated number of particles, a relative value of the amount of CD63 per number was calculated using the value calculated from the "recovered fraction" obtained from "2,000 × g-treated supernatant sample" of MagCapture as a control, and the results are shown in Table 5.

**[Table 5]**

| | CD63 signal | Number of particles (× 10¹⁰/mL) | CD63 corrected by number of particles | Relative value of amount of CD63 per number |
|---|---|---|---|---|
| MagCapture | 0.36 | 7.09 | 0.051 | 1.00 |
| 40/30000 | 0.38 | 4.03 | 0.095 | 1.89 |
| 40/10000 | 0.20 | 1.27 | 0.160 | 3.18 |
| 40/1000 | 0.08 | 0.49 | 0.169 | 3.35 |

It has been reported that amount of CD markers (CD63 and the like) per number of particles is higher in small EV having a smaller particle diameter than in large EV (Large and small extracellular vesicles released by glioma cells in vitro and in vivo, Journal of Extracellular Vesicles, volume 9, issue 19, 2020). From Table 5, it was suggested that, as compared with MagCapture having no exclusion limit molecular weight, the CD63 signal corrected by the number of particles was high in "40/30000", "40/10000", and "40/1000", which have an exclusion limit molecular weight, and since the amount of CD63 per number increased as the exclusion limit molecular weight decreased, the small EV was easily purified by the exclusion limit molecular weight in "40/30000", "40/10000", and "40/1000".

### (Comparison of EV capture rate)

The EV capture rate was compared by the following operation.

100 µL of each of WorkBeads 40/100 ACT, 40/1000 ACT, 40/10000 ACT, and 40/30000 ACT (all manufactured by Bio-Works) was suspended in 1 mL of a phosphate buffer (pH 7.4) to obtain a suspension. 4 µg of Tim4-Fc protein (FUJIFILM Wako Pure Chemical Corporation #137-18511) was added to each of the above-described suspensions, and the mixture was shaken (1,400 rpm) at 4°C for 24 hours. The mixture was subjected to centrifugal treatment at 2,000 × g for 1 minute, the supernatant was removed, and beads were obtained. Each of the obtained beads was washed three times with 500 µL of TBS and then the beads were resuspended in 200 µL of TBS and incubated at room temperature for 1 hour or more. 1 mL of the above-described "0.22 µm filter-treated supernatant sample" was added to 10 µL of each of the Tim4-immobilized beads after incubation, and the mixture was mixed by inversion at room temperature for 2 hours. The beads were precipitated by centrifugal treatment at 2,000 × g for 1 minute, and each of the supernatant "unrecovered fraction" was recovered. The precipitated beads were washed twice with 400 µL of 0.0005% Tween 20 + 2 mM CaCl₂/TBS, and then loaded on a 0.45 µm filter, and subjected to centrifugal treatment at 400 × g for 1 minute to remove the residual liquid. "An operation of adding 50 µL of 10 mM EDTA + 1% EV-save for in vivo/TBS to each bead, allowing the mixture to stand at room temperature for 10 minutes, and recovering the eluate by a centrifugal treatment at 400 × g for 1 minute" was performed twice, the eluates recovered twice was mixed, and the mixture was referred to as a "recovered fraction".

In addition, the "unrecovered fraction" and "recovered fraction" obtained by using the "0.22 µm filter-treated supernatant sample" were recovered by the same method as the "EV purification from MSC supernatant by MagCapture Exosome Isolation Kit PS" described above.

Each of the "0.22 µm filter-treated supernatant sample" and "unrecovered fraction" was subjected to operation using PS Capture Exosome ELISA kit (Streptavidin HRP) (FUJIFILM Wako Pure Chemical Corporation #298-80601) according to the protocol. Each of the absorbance was measured with a plate reader Spark (manufactured by Tecan Group Ltd.). The proportion (FT signal) of each "unrecovered fraction" to the "0.22 µm filter-treated supernatant sample" was calculated, and the capture rate was calculated by (1 - FT signal) × 100, which are shown in Table 6.

**[Table 6]**

| | Capture rate (%) |
|---|---|
| MagCapture | 99.9 |
| 40/30000 | 76.9 |
| 40/10000 | 24.3 |
| 40/1000 | 17.5 |
| 40/100 | -2.6 |

From Table 6, it can be seen that MagCapture does not have an exclusion limit molecular weight, and thus most of the particles can be captured. On the other hand, in the WorkBeads, the EVs that can be captured are limited by the exclusion limit molecular weight, and thus the capture rate is decreased. In WorkBeads 40/100, the capture rate was negative, which indicated that EV could not be captured. This suggests that the exclusion limit molecular weight is too small compared to the size of EV, and thus any population of EV cannot be captured.

### (Measurement of micropore diameter by small angle X-ray scattering method (SAXS))

For each of WorkBeads 40/100 ACT, 40/1000 ACT, 40/10000 ACT, and 40/30000 ACT, small angle X-ray scattering was measured under the following conditions, and the average micropore diameter was calculated. The median diameter of each bead and the proportion (%) of micropores having a micropore diameter of 20 nm or more with respect to all micropores are shown in Table 7.
Device: Nanopix (Cu Kα, 2PUHR, camera length of 2000 mm) manufactured by Rigaku Corporation
Optical system: 2PUHR, camera length of 1100 mm
Specimen: the specimen is used by being sealed in a φ1 mm glass capillary in the form of a stock solution
Measurement: integration of 10 minutes × 2 times (sequential accumulation) at normal temperature and normal pressure
Analysis: the particle size distribution at q < 0.46 was calculated by fitting using the maximum entropy method (particle shape: sphere).

**[Table 7]**

| | Median diameter (nm) | Proportion of micropore diameter of 20 nm or more (%) | Exclusion limit molecular weight (kDa) |
|---|---|---|---|
| 40/30000 | 20.3 | 33 | 30,000 |
| 40/10000 | 20.2 | 32 | 10,000 |
| 40/1000 | 18 | 25 | 1200 |
| 40/100 | 15.9 | 22 | 150 |

From Table 7, it can be seen that the exclusion limit molecular weight is correlated with the micropore diameter (median diameter) and the proportion of the micropore diameter of 20 nm or more. In 40/100, it is considered that since the EV cannot be purified, it is necessary to have at least the median diameter or the proportion of the micropore diameter of 20 nm or more, which is indicated by 40/100. It is known that a small EV generally has a micropore diameter of 20 nm to 200 nm (Journal of EXTRACELLULAR VESICLES, Volume 11, Issue 10 (October 2022)), but it was unexpected that a small EV could enter the micropore of the carrier according to the embodiment of the present invention and could be captured and purified, in consideration of the median diameter and the proportion of the micropore diameter of 20 nm or more, which is shown in the above table.

### (Examination of purification efficiency based on change in immobilization amount of Tim4)

By the following operation, the purification efficiency due to the change in the immobilization amount of Tim4 was examined.

100 µL of each of WorkBeads 40/30000 ACT (Bio-Works) was suspended in 1 mL of a phosphate buffer (50 mM, pH 7.4), and 4, 8, 16, 32, 64, 128, or 256 µg of Tim4-Fc protein (FUJIFILM Wako Pure Chemical Corporation #137-18511) was added thereto. After shaking at 4°C for 24 hours (1,400 rpm), centrifugal treatment was performed at 2,000 × g for 1 minute and the supernatant was recovered ("non-immobilized fraction"). The beads were washed with TBS three times, re-suspended in TBS, and incubated at room temperature for 1 hour or more. The operation was performed in the same manner as in the above-described "EV purification from MSC supernatant by preparing Tim4-immobilized beads", and the "unrecovered fraction" and "recovered fraction" of the above-described "0.22 µm filter-treated supernatant sample" were recovered. The recovered "unrecovered fraction" and "recovered fraction" were subjected to operation according to the protocol using a biotin-conjugated anti-CD9 antibody (FUJIFILM Wako Pure Chemical Corporation # 013-27951) in a PS Capture Exosome ELISA kit (Streptavidin HRP) (FUJIFILM Wako Pure Chemical Corporation #298-80601). The proportion (FT signal) of the "unrecovered fraction" and the proportion (EV signal) of the "recovered fraction" to the "0.22 µm filter-treated supernatant sample" were calculated. In addition, the capture rate was calculated by (1 - FT signal) × 100.

The results of measuring the capture rate are shown in Table 8 below. It is noted that since 100 µL of each of WorkBeads 40/30000 ACT (Bio-Works) was used, in a case where 4, 8, 16, 32, 64, 128, or 256 µg of the Tim4-Fc protein was added, each of the concentration was 40, 80, 160, 320, 640, 1280, or 2560 µg/mL.

**[Table 8]**

| | 40 µg/mL | 80 µg/mL | 160 µg/mL | 320 µg/mL | 640 µg/mL | 1,280 µg/mL | 2,560 µg/mL |
|---|---|---|---|---|---|---|---|
| Capture rate (%) | 96.5 | 99.9 | 100.0 | 100.0 | 99.9 | 99.7 | 98.5 |
| Elution rate (%) | 71.9 | 74.2 | 82.4 | 65.6 | 66.3 | 47.4 | 33.5 |

From Table 8, the elution rate was significantly reduced as the immobilization amount increased with 160 µg/mL as the apex. On the other hand, no change in the capture rate was confirmed. The recovery rate was lower than 50% in a case where the immobilization amount was 1,280 µg/mL or more. The recovery rate of 70% was achieved in a case where the lower limit was 40 µg/mL or more. From the above results, it was suggested that in the affinity purification with Tim, more efficient purification can be performed by setting a specific immobilization amount (40 to 1,000 µg/mL carrier = 33.5 to 966.2 µg/g carrier (calculated by the density shown in Table 9)).

### (Measurement of density of agarose beads)

The density of the beads was measured by the following method.

1 mL of 50% slurry WorkBeads 40/1000, WorkBeads 40/10000, and WorkBeads 40/30000 were subjected to centrifugal treatment at 10,000 ^{x} g for 2 minutes to precipitate beads. 500 µL of the supernatant was removed with a pipette, and the mass was measured and converted into a mass per 1 mL.

The measurement results are shown in Table 9 below.

**[Table 9]**

| | Density (g/mL) |
|---|---|
| 40/30000 | 1.0974 |
| 40/10000 | 1.035 |
| 40/1000 | 1.1956 |

## Claims

1. A porous carrier that is for acquiring extracellular vesicles and has an exclusion limit molecular weight of 800 to 60,000 kDa, to which a substance having an affinity for extracellular vesicles is bound.

2. The porous carrier according to claim 1,
wherein the exclusion limit molecular weight is 1,500 to 35,000 kDa.

3. The porous carrier according to claim 1,
wherein pores included in the porous carrier is non-penetrating pores.

4. The porous carrier according to claim 1,
wherein a median diameter of pores included in the porous carrier is 17 to 21 nm.

5. The porous carrier according to claim 1,
wherein the substance having an affinity for extracellular vesicles is a phosphatidylserine-binding substance.

6. The porous carrier according to claim 5,
wherein the phosphatidylserine-binding substance is a Tim protein.

7. The porous carrier according to claim 1,
wherein the porous carrier is a porous particle or a porous membrane.

8. The porous carrier according to claim 1,
wherein a binding amount of the substance having an affinity for extracellular vesicles with respect to 1 g of the porous carrier is 1,000 µg/g or less.

9. The porous carrier according to claim 1,
wherein the porous carrier is composed of polysaccharides.

10. The porous carrier according to claim 1,
wherein a proportion of a quantity of extracellular vesicles having a particle diameter of 200 nm or less is 85% or more in the entire acquired extracellular vesicles.

11. A method for acquiring extracellular vesicles in a specimen using the porous carrier according to any one of claims 1 to 10.

12. A method for producing extracellular vesicles, comprising:
bringing a specimen containing extracellular vesicles into contact with the porous carrier according to any one of claims 1 to 10 to form a complex of a substance having an affinity for extracellular vesicles bound to the porous carrier and the extracellular vesicles in the specimen;
separating the complex from the specimen; and
separating the extracellular vesicles from the complex to acquire the extracellular vesicles.

13. A method for producing extracellular vesicles, comprising:
bringing a specimen containing extracellular vesicles into contact with the porous carrier according to any one of claims 1 to 10 to form a complex of a substance having an affinity for extracellular vesicles bound to the porous carrier and the extracellular vesicles in the specimen; and
removing the complex.

14. A kit for acquiring extracellular vesicles, comprising:
a porous carrier having an exclusion limit molecular weight of 800 to 60,000 kDa; and
a substance having an affinity for extracellular vesicles.
